# EUROPEAN PATENT APPLICATION

(11) **EP 2 508 168 A1**
(43) Date of publication of application: **10.10.2012**
(21) Application number: 12155566.8
(22) Date of filing: 15.02.2012
(51) Int. Cl.: A61K 8/87, A61Q 5/06

(54) **Cosmetic composition comprising polymer comprising direct crosslinks**

(30) Priority: 06.04.2011 EP 11161294
(71) Applicant: The Procter & Gamble Company, Cincinnati, OH 45202 (US)
(72) Inventor: Flohr, Andreas, 61476 Kronberg im Taunus (DE); Kripp, Thomas, 64407 Fränkisch-Crumbach (DE)
(74) Representative: Sievers, Uwe

(57) **Abstract**

A composition comprising polymer P^{x}, wherein polymer P^{x} comprises a moiety of formula selected from the group consisting of: Formula 1, Formula 2 and a combination of Formula 1 and Formula 2: 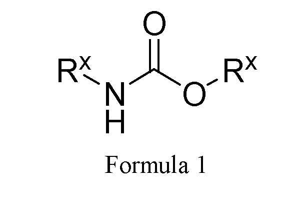 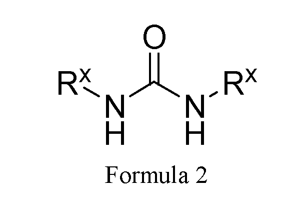 wherein R^{x} comprises a group according to Formula 3: 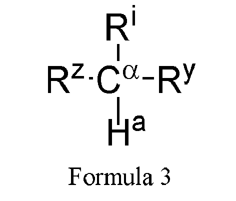 wherein R^{y} and R^{z} are, independently, any suitable atom or moiety; and Rⁱ is an electron-withdrawing functional group; and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom. Also *inter alia* a cosmetic composition, a hairstyling composition, a method of styling hair, the use of the composition, a kit and a process for crosslinking.

## Description

### FIELD OF THE INVENTION

According to one aspect, the invention relates to a composition comprising polymer P^{x}, wherein polymer P^{x} comprises *inter alia* at least one direct crosslink, wherein the direct crosslink is a direct covalent bond between the C^{α} of the polymer P^{x} and a second C^{α} of a polymer P^{x}, and wherein said direct crosslink comprises no intermediary atom. According to other aspects, the invention relates *inter alia* to a cosmetic composition, a hairstyling composition, a method of styling hair, the use of the composition, a kit and a process for crosslinking.

### BACKGROUND OF THE INVENTION

Cosmetic compositions are commonly used for treating the skin and/or keratinous materials, e.g. hair, of the users. A common ingredient of cosmetic compositions is polymer-based ingredients. Various polymers, with a different properties and providing a range of benefits, are suitable for incorporation into cosmetic compositions. Extensive information about marketed polymers is listed in the International Cosmetic Ingredient Dictionary and Handbook, 12^{th} edition (2008) from the Personal Care Products Council (formerly the Cosmetic, Toiletry and Fragrance Association or CTFA). For example, polymers may be emulsion stabilizers, suspending agents, viscosity increasing agents, opacifying agents, film-forming agents, hair-fixative agents, skin moisturizing agents and/or hair conditioning agents.

Depending on the class of monomers, their structure, degree of saturation and/or neutralisation, proportions in the polymer, and/or the polymerization process, polymers exhibit specific properties and provide specific benefits. In order to customize further polymer properties and/or in order to render polymers more suitable for targeted applications, these polymers may be modified further, e.g. by grafting chemical groups and/or by crosslinking the polymeric backbone using crosslinking agents.

A conventional crosslinking process consists of forming a crosslink between two crosslinkable units using a crosslinking agent. Crosslinking agents are at least bi-functional compounds which form, usually upon activation, a bridge between two moieties i.e. two bonds are formed resulting in: moiety A-crosslinker-moiety B. These crosslinks are known hereinafter as "indirect crosslinks".

Concerning the synthesis of these indirect crosslinks, there are two common methods employed. The first is *in situ* crosslinking, which occurs during the polymerisation reaction. This is typically done by adding e.g. a dimeric crosslinking agent into the polymerisation reaction mixture. An example of a conventional crosslinking agent used for polymers used in cosmetic applications is methylene-*bis*-acrylamide, of formula (CH₂=CHCONH)₂CH₂, which is particularly suitable for forming indirect crosslinks between two moieties derived from acrylamide monomers. Other conventional crosslinking agents include: di(meth)acrylate, *N*-(1-hydroxy-2,2-dimethoxyethyl)acrylamide, ethyleneglycol dimethacrylate, ethyleneglycol diacrylate, allylmethacrylate, 1,1,1-trimethylolpropane triacrylate, triallylamine, and tetraallyloxethane. The second method is carried out after derivatisation of reactive groups in the polymer. For example, in case of the presence of acidic groups as present in polyacrylic acid, it is possible to form diester bridges by means of diol crosslinking agents or diamide bridges by the help of diamine crosslinking agents.

Indirect crosslinking of the polymer alters its properties, e.g. its water-solubility, molecular weight, glass transition temperature, consistency, elasticity, melting behaviour, and porosity. The new properties of the crosslinked polymer depends on various factors, e.g. on the size and chemistry of the crosslinking agent, the density of crosslinks, and whether inter-strand or intra-strand crosslinks are formed. In the context of polymers, when two moieties from different polymeric backbones are crosslinked to each other, an inter-strand crosslink is obtained. When two moieties comprised within the same polymeric backbone are crosslinked to each other, an intra-strand crosslink is obtained. Bridges are typically based on ester, ether or amide bonds and are prone to cleavage by hydrolysis. This is a drawback, because of the instability of the indirect crosslinks, especially in aqueous media. Therefore, there is a need for providing crosslinks that are more stable.

A further drawback of indirect crosslinking is that it necessitates the presence of specific functional groups in the polymer, which are compatible with the chosen crosslinking agent. However, the resultant indirectly crosslinked polymer may have undesired chemistry or physical characteristics. Furthermore, there is a restriction in the number of sites available to the crosslinking agent to react with and these sites should not be sterically hindered from the crosslinking agent or have already reacted with another ingredient of the reaction mixture. Consequently, the polymer to be indirectly crosslinked and the crosslinking agent have to be very carefully selected, in addition to their proportions and the reaction conditions such that an appropriate density of crosslinks occurs. Therefore, there is a need for providing a means to crosslink a wider variety of polymers such that their resultant chemistry and properties meet cosmetic needs.

Indeed, crosslinking usually alters the elastic properties of polymers. In general, a higher crosslinking density increases elasticity, although a too high crosslinking density may render the crosslinked polymer too brittle. The provision of polymers exhibiting increased rigidity/stiffness, increased brittleness or reduced elasticity is however not preferred for incorporation into cosmetic compositions as it impacts on the properties of the composition, its ease of application onto skin and/or hair and on the benefits conferred to skin and/or hair. Instead, it is preferred to use elastomeric polymers, i.e. polymers having suitable elastic properties. Indeed, there is a need for improving the elastic properties of commercially available polymers suitable for cosmetic purposes.

Hairstyling compositions are expected to provide satisfactory hold and setting properties to a hairstyle. However, hairstyling compositions comprising currently available hairstyling polymers confer long-lasting hold and high setting properties to the hairstyle, but a limited elasticity, if any. There is a need therefore for improving the elastic properties of commercially available hairstyling polymers. There is also a need for providing a hairstyling polymer capable of imparting excellent hold to a hairstyle and also excellent flexibility and bounce to the hairstyle.

Moreover, a known problem for consumers utilising hairstyling products is hairstyle sag and deterioration over time requiring the need to refresh the hairstyle by reapplying more hairstyling product and re-shaping the hairdo. Typically consumers create the hairstyle at home in the morning, but may need to refresh the hairstyle on multiple occasions throughout the day. Moisture content of the environment is an important factor in how regularly consumer need to refresh the hairdo during the day i.e. high environmental humidity causes faster hairdo sag and deterioration. Therefore, there is a need for providing hairstyling products that can be applied less frequently and result in a durable hairdo that is also resistant to humidity and moisture.

Furthermore, there is a need for hairstyling products that can be used by consumers at home on their own hair as well as by skilled professional hair stylists in a salon environment. In other words, the efficacy of the product should meet the little trained skill of an at-home consumer who typically create their own hairstyle with the aid of their own hands and rudimentary equipment such as mirrors and blow dryers. Furthermore, the product should also meet the requirements of a trained hairdresser or hair stylist, in a well-equipped salon, that needs to create fresh, trendy hairstyle that the consumer is not able to create at home.

Alternative crosslinking processes are already known in different technical fields. See, e.g. EP1568385A1, which relates to superabsorbent polymers comprising direct crosslink between polymer chain segments. These superabsorbant materials are utilised in absorbent articles, such as nappies, training pants, adult incontinence products, and feminine care products to increase the absorbent capacity of such products while reducing their overall bulk. There is a need, however, for providing cosmetic compositions comprising crosslinked polymers being obtained via an alternative process to conventional crosslinking using crosslinking agents.

### SUMMARY OF THE INVENTION

According to one aspect, the present invention relates to a composition comprising polymer P^{x}, wherein polymer P^{x} comprises a moiety of formula selected from the group consisting of: Formula 1, Formula 2 and a combination of Formula 1 and Formula 2, as shown hereinafter; wherein R^{x} comprises a group according to Formula 3, as shown hereinafter; wherein R^{y} and R^{z} are, independently, any suitable atom or moiety; and Rⁱ is an electron-withdrawing functional group; and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

According to another aspect, the present invention relates to a method of styling hair comprising:
(a) providing a composition according to the first aspect;
(b) applying said composition to hair;
(c) styling the hair.

According to another aspect, the present invention relates to the use of a composition, as detailed herein, for styling hair.

According to another aspect, the present invention relates to a kit comprising:
(a) a cosmetic formulation comprising:
   i. a polymer P comprising a moiety of formula selected from the group consisting of: Formula 1, Formula 2, and a combination of Formula 1 and Formula 2, as shown hereinafter;
      wherein R^{x} comprises a group according to Formula 3 as shown hereinafter;
      and wherein R^{y} and R^{z} are, independently, any suitable atom or moiety; and Rⁱ is an electron-withdrawing functional group;
   ii. a cosmetically acceptable carrier;
(b) a photoinitiator;
wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

According to another aspect, the present invention relates to a method of styling hair comprising:
I. providing the kit, as detailed herein;
II. mixing the cosmetic formulation with the photoinitiator resulting in a mixed cosmetic formulation;
III. irradiating said mixed cosmetic formulation with UV resulting in an irradiated cosmetic formulation;
IV. applying the irradiated cosmetic formulation to hair;
V. styling the hair;
VI. exposing the hair to a temperature of from about 20°C to about 80°C, preferably from about 30°C to about 60°C, more preferably from about 35°C to about 55°C, even more preferably from about 40°C to about 50°C.

According to another aspect, the present invention relates to a process comprising:
(a) the provision of a formulation comprising a polymer P, wherein polymer P comprises a moiety of formula selected from the group consisting of: Formula 1, Formula 2, and a combination of Formula 1 and Formula 2, as shown hereinafter; wherein R^{x} comprises a group according to Formula 3, as shown hereinafter; and wherein R^{y} and R^{z} are, independently, any suitable atom or moiety; and Rⁱ is an electron-withdrawing functional group;
(b) the provision of a photoinitiator;
(c) mixing said polymer P with said photoinitiator resulting in a mixed formulation;
(d) the irradiation of said mixed formulation with UV to form at least one direct crosslink, wherein the direct crosslink is a direct covalent bond between the C^{α} of the polymer P and a second C^{α} of a polymer P, and wherein said direct crosslink comprises no intermediary atom.

According to another aspect, the present invention relates to the use of a kit, as detailed herein, for styling hair.

### BRIEF DESCRIPTION OF THE DRAWINGS

Fig. 1: Wet curl retention test - crosslinked versus uncrosslinked.
Fig. 2: Wet curl retention test - duration of UV treatment.
Fig. 3: Wet curl retention test - amount of cationic surfactant.

### DETAILED DESCRIPTION OF THE INVENTION

All percentages, ratios and proportions herein are by weight, unless otherwise specified. All percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements.

As used herein, the expressions "weight percentage" and "percentage by weight" of a component mean the percentage of active component and not the percentage of the raw material comprising this active, unless otherwise specified.

All numerical amounts are understood to be modified by the word "about" unless otherwise specifically indicated. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or byproducts that may be included in commercially available materials, unless otherwise specified.

The term "molecular weight" or "M.Wt." as used herein refers to the number average molecular weight unless otherwise stated.

Herein, "comprising" means that other steps and other ingredients which do not affect the end result can be added. This term encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein.

The term "substantially free from" or "substantially free of' as used herein means less than about 1%, preferably less than about 0.8%, more preferably less than about 0.5%, still more preferably less than about 0.3%, most preferably about 0%, by total weight of the composition or formulation.

As used herein, the term "elastomeric" means a group of polymers, which are crosslinked to only a certain degree in order to provide them with elasticity without making them too stiff. Said polymers usually are characterized with a glass transition temperature below room temperature. Said elastomers are classified as an intermediary category between non-crosslinked linear polymers and totally crosslinked polymers (e.g. thermosets).

As used herein, the term "UV" and the equivalent terms "ultra-violet", "UV", "UV light" and "UV radiation", means radiation with a wavelength of between 400 nm and 10 nm.

As used herein, the term "photoinitiator" means an agent which initiates a free radical-based reaction upon exposure to UV. The photoinitiator may reactant with UV such that at least one free radical is formed. The radical may abstract a hydrogen radical in an alpha position relative to the electron-withdrawing group.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivative," as used herein, includes but is not limited to, amide, ether, ester, amino, carboxyl, acetyl, and/or alcohol derivatives of a given compound.

"Monomer," as used herein, means a discrete, non-polymerised chemical moiety capable of undergoing polymerisation in the presence of an initiator.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

As used herein, the term "C^{α}" and the equivalent terms "alpha-carbon", "carbon in an alpha position", mean carbon atom directly attached to the relevant functional group. C^{α} may mean a carbon directly attached to an electron-withdrawing group.

As used herein, the term "electron-withdrawing group" and the equivalent term "electron-withdrawing functional group", mean a chemical moiety with a greater electron-withdrawing capability than hydrogen.

As used herein, the term "H^{α}" and the equivalent terms "alpha-hydrogen", "hydrogen in an alpha position", "H^{α}", "abstracted hydrogen" mean a hydrogen atom directly attached to a C^{α} in an alpha position relative to the electron-withdrawing group. H^{a} may be abstracted from the C^{α} following reaction with an activated photoinitiator.

As used herein, the term "moiety" means a group of bonded atoms, for example forming a functional group, a part of a functional group, a group of functional groups, a part of a molecule or an entire molecule.

As used herein, the term "moiety of formula X" and the equivalent terms "chemical moiety of formula X", "chemical moiety of chemical formula X", "chemical moiety of formula X" means a moiety with a chemical structure complying with and/or corresponding to the formula or formulas named thereafter.

As used herein, the term "polymer molecule" means a group of atoms, comprising a single polymer backbone, connected by covalent bonds. A polymer molecule may be branched.

As used herein, the term "crosslinked" means directly crosslinked, unless stated otherwise.

"Kit," as used herein, means a packaging unit comprising a plurality of components. An example of a kit is, for example, a first composition and a separately packaged second composition. Another kit may comprise a first composition and an energy delivery device. A different kit may comprise three different types of separately packaged composition and a hair styling implement. A further kit may comprise application instructions comprising a method and a composition/formulation.

"Separately packaged," as used herein, means any form of packaging that prevents a first composition from coming into physical contact, or admixing, with a second composition. "Separately packaged" may mean that the individual compositions are packaged in separate containers, or alternatively in a single container partitioned such that the compositions are not in physical contact.

"Implement," as used herein, means a device used to facilitate application of a composition to the hair and/or manipulation of the hair. Examples of implements include, but are not limited to, a comb, a means for directed delivery (e.g., an applicator or tube), a covering for the hair (e.g., plastic bag, shower cap, etc.), and combinations thereof.

The term "hairstyling polymer" as used herein means hair-fixing polymers which form films on a surface. In the context of hair, this surface is the surface of individual hair fibres or a plurality thereof. The polymer causes them to be glued together to build welds, which are crosslinks that provide the hold benefit. In concert, these welds form a 'hairnet' to provide hair hold and volume benefits to the user. When the net of welds is effectively formed, the hold and volume benefits can last all day and offer good resistance to environmental humidity.

According to one aspect, the present invention relates to a composition comprising polymer P^{x}, wherein polymer P^{x} comprises a moiety of formula selected from the group consisting of: Formula 1, Formula 2 and a combination of Formula 1 and Formula 2: 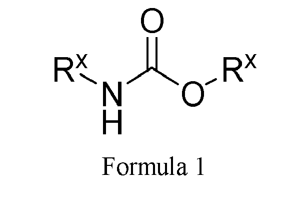 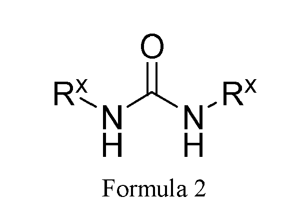 wherein R^{x} comprises a group according to Formula 3: 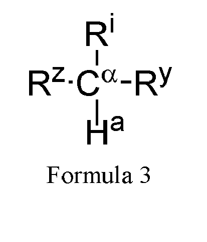 wherein R^{y} and R^{z} are, independently, any suitable atom or moiety; and Rⁱ is an electron-withdrawing functional group;
and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

The inventors have surprisingly found that a polymer P^{x} comprising direct crosslinks exhibits an excellent balance between improved elastic properties and hold of hairstyle. Thus, hair treated with a composition comprising polymer P^{x} is able to hold a desired hairstyle, but without imparting stiffness and rigidity to the hair i.e. the hair retains flexibility and bounce. Furthermore, a polymer P^{x} provides improved resistance to wetness, including environmental humidity.

Without being bound by any theory, it is believed that the direct crosslinking technology, as described herein, results in a polymer P^{x} comprising crosslinks being C-C bonds. In order to form such direct crosslinks, the two H^{a}-C^{α}-Rⁱ moieties are required, wherein Rⁱ is an electron-withdrawing group that allows a photoinitiator, following UV activation, to abstract a hydrogen radical H^{a}. This reaction results in a C˙ radical, which is able to form a C-C bond when the first C˙ radical is in proximity to a second C˙ radical. The crosslinks being direct C-C bonds between crosslinked moieties has two main advantages. Firstly, the C-C bond is much more stable than indirect crosslinks, which are typically ester, amide or ether bonds. Therefore, the crosslinking is much less likely to be degraded, e.g. by chemicals present in other hairstyling products, shampoos, conditioners, heat from blow drying or flat irons, and other hair products and treatments.

Furthermore, due to the high frequency of electron-withdrawing groups and C-H bonds present in common cosmetic compounds, e.g. hairstyling polymers, H^{a}-C^{α}-Rⁱ moieties are relatively abundant. This means that there are many sites for direct crosslinks within the compound. Furthermore the direct crosslinks can be more evenly distributed more evenly throughout the polymer.

Moreover, the utilisation of UV in order to activate the photoinitiator means that the direct crosslinking is easy to achieve and tailor. For example, it is possible to simply alter the ratio of the photoinitiator to the polymer P, and the potency of the UV and duration of the irradiation, and thus vary the density of the crosslinks.

In addition, the inventors have found that it is possible to dispense with the need for UV irradiating hair pre-coated with a formulation comprising uncrosslinked polymer P and a photoinitiator. Without being bound by theory, it is believed that the mechanism for the formation of the direct crosslinks is as follows: (1) activation of the photoinitiator via UV irradiation - radicals are formed; (2) abstraction of a hydrogen H^{a} from the polymer P, when in the context of a H^{a}-C^{α}-Rⁱ moiety, by the photoinitiator radical resulting in a C˙ radical; (3) the reaction of two C˙ radicals in the presence of oxygen resulting in a labile peroxo-bridge (C^{α}-O-O-C^{α}); (4) conversion of the peroxo-bridge to a C^{α}-C^{α}. It is believed that step 4 occurs naturally over time, but the conversion can be sped up by the application of heat. Thus excellent performance results from a method wherein a formulation comprising uncrosslinked polymer P and a photoinitiator is UV irradiated, subsequently applied onto hair and then heat is applied in order to convert any remaining peroxo-bridges into C^{α}-C^{α} direct crosslinks.
The inventors have also surprisingly found that cosmetic compositions - e.g. skin and/or hair care compositions, skin and/or hair cleansing compositions, hair styling compositions, hair conditioning compositions, hair dyeing compositions and/or shaving compositions - incorporating crosslinked polymer P^{x} shows an improved performance, particularly vis-à-vis the physical properties, the ease of application and the benefits conferred to the skin and/or hair. Crosslinked polymer P^{x} is particularly suitable for incorporation into hair styling compositions. Said compositions confer long-lasting hold and high-setting properties, together with elasticity to the hair style.

Polymer P^{x} is a polymer comprising a specific chemical moiety and at least one direct crosslink. In all embodiments herein, polymer P^{x} is polymer P, as described herein, further comprising at least one direct crosslink, preferably a plurality of direct crosslinks.
Polymer P^{x} and polymer P comprise R^{x}, which comprises R^{y} and R^{z}. R^{y} and R^{z} are, independently, any suitable atom or moiety. Suitable atoms or moieties include atoms and moieties suitable for inclusion in a polymer suitable for cosmetic purposes and not rendering the polymer incapable of forming or retaining direct crosslinks. In an embodiment, R^{y} and R^{z} are, independently, selected from the group consisting of: any organic atom or organic moiety; hydrogen; a saturated aliphatic group; alkyl group comprising 1 to 20 carbon atoms; Rⁱ; and mixtures thereof. In an embodiment, at least one of either R^{y} or R^{z} is hydrogen.

The polymer P^{x} may relate to "a combination of Formula 1 and Formula 2". Suitable combinations of Formula 1 and Formula 2 include: Formula J, Formula K, and Formula L, which are shown below. Formula J: 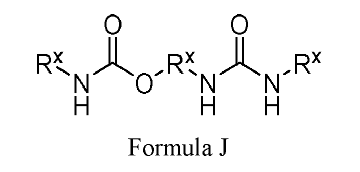 wherein R^{x} comprises a group according to Formula 3, as shown above;
wherein R^{y} and R^{z} are any suitable atom, molecule or group; and Rⁱ is an electron- withdrawing functional group. Formula K: 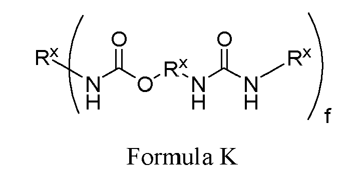 wherein f is at least 1, preferably from 4 to 40; wherein R^{x} comprises a group according to Formula 3, as shown above; wherein R^{y} and R^{z} are, independently, any suitable atom or moiety; and Rⁱ is an electron-withdrawing functional group. Formula L: 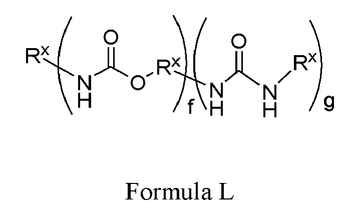 wherein f is at least 1, preferably from 4 to 40; and wherein g is at least 1, preferably from 4 to 40; wherein R^{x} comprises a group according to Formula 3, as shown above; wherein R^{y} and R^{z} are any suitable atom, molecule or group; and Rⁱ is an electron- withdrawing functional group.

An embodiment relates to a composition comprising polymer P^{x}, wherein polymer P^{x} comprises a moiety of formula selected from the group consisting of Formula 4 and Formula 5: 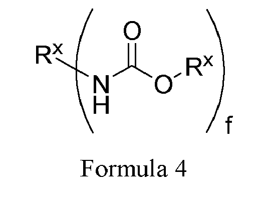 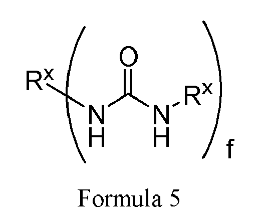 wherein R^{x} comprises a group according to Formula 3, as shown above;
wherein R^{y} and R^{z} are, independently, any suitable atom or moiety; and Rⁱ is an electron-withdrawing functional group;
and wherein f is from 4 to 40;
and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

An embodiment relates to a composition comprising polymer P^{x}, wherein polymer P^{x} comprises a moiety of formula selected from the group consisting of Formula 4, as shown above; wherein R^{x} comprises a group according to Formula 3, as shown above; wherein R^{y} and R^{z} are any suitable atom or moiety; Rⁱ is an electron-withdrawing functional group; and f is from 2 to 40, or 4 to 40, or from 5 to 40, or from 10 to 40;
and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

An embodiment relates to a composition comprising a dispersed polyurethane comprising a polymer P^{x} which is the reaction products of:
A) a prepolymer according to the Formula PP: 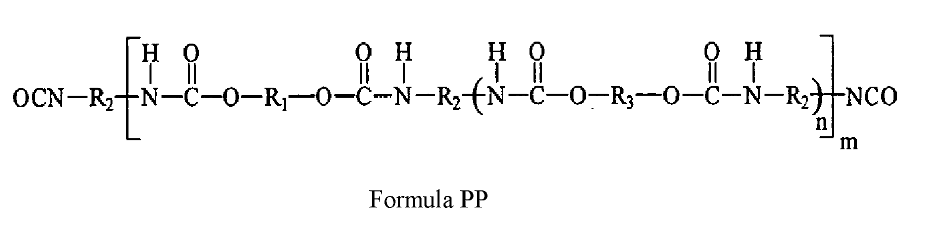 wherein
   R₁ represents a bivalent radical of a dihydroxyl functional compound, R₂ represents a hydrocarbon radical of an aliphatic or cycloaliphatic polyisocyanate, R₃ represents a radical of a low molecular weight diol, optionally substituted with ionic groups,
   n is from 0 to 5, and m is >1;
B) at least one first chain extender according to the formula: H₂N-R₄-NH₂ wherein R₄ represents an alkylene or alkylene oxide radical not substituted with ionic or potentially ionic groups; and
C) at least one second chain extender according to the formula: H₂N-R₅-NH₂ wherein R₅ represents an alkylene radical substituted with ionic or potentially ionic
groups;
wherein at least one of R₁, R₂ and R₃ comprises a group according to Formula 3, as shown above; wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

Suitable dihydroxyl compounds for providing the bivalent radical R₁ are those having two hydroxy groups and having number average molecular weights of from about 700 to about 16,000, and preferably from about 750 to about 5000. Examples of the high molecular weight compounds are polyester polyols, polyether polyols, polyhydroxy polycarbonates, polyhydroxy polyacetals, polyhydroxy polyacrylates, polyhydroxy polyester amides, polyhydroxy polyalkadienes and polyhydroxy polythioethers. The polyester polyols, polyether polyols and polyhydroxy polycarbonates are preferred. Mixtures of various polyols are also within the scope of the present invention.

Polyester polyols include polyester diols. The polyester diol(s) may be prepared in known manner from aliphatic, cycloaliphatic or aromatic dicarboxylic or polycarboxylic acids or anhydrides thereof (for example, succinic, glutaric, adipic, pimelic, suberic, azelaic, sebacic, nonanedicarboxylic, decanedicarboxylic, terephthalic, isophthalic, o-phthalic, tetrahydrophthalic, hexahydrophthalic or trimellitic acid) as well as acid anhydrides (such as o-phthalic, trimellitic or succinic acid anhydride or a mixture thereof) and dihydric alcohols such as, for example, ethanediol, diethylene, triethylene, tetraethylene glycol, 1,2-propanediol, dipropylene, tripropylene, tetrapropylene glycol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, 1,8-octanediol, 1,10-decanediol, 1,12-dodecanediol or mixtures thereof. Cycloaliphatic and/or aromatic dihydroxyl compounds are, of course, also suitable as the dihydric alcohol(s) for the preparation of the polyester polyol(s). The corresponding polycarboxylic acid anhydrides or corresponding polycarboxylic acid esters of low alcohols, or mixtures thereof, may also be used in place of the free polycarboxylic acid for the preparation of the polyesters.

The polyester diols may naturally also be homopolymers or copolymers of lactones, which are preferably obtained by addition reactions of lactones or lactone mixtures, such as butyrolactone, ε-caprolactone and/or methyl-ε-caprolactone with the suitable difunctional starter molecules such as, for example, the low molecular weight dihydric alcohols mentioned above. The corresponding polymers of ε-caprolactone are preferred.

Polycarbonates containing hydroxy groups include those known per se such as the products obtained from the reaction of diols such as propanediol-(1,3), butanediol-(1,4) and/or hexanediol-(1,6), diethylene glycol, triethylene glycol or tetraethylene glycol with diarylcarbonates, e.g. diphenylcarbonate or phosgene.

Suitable polyether polyols are obtained in known manner by the reaction of starting compounds which contain reactive hydrogen atoms with alkylene oxides such as ethylene oxide; propylene oxide; butylene oxide; styrene oxide; tetrahydrofuran or epichlorohydrin or with mixtures of these alkylene oxides. It is preferred that the polyethers do not contain more than about 10% by weight of ethylene oxide units. Most preferably, polyethers obtained without the addition of ethylene oxide are used.

Suitable starting compounds comprising reactive hydrogen atoms include, e.g. water and the dihydric alcohols described above for preparing the polyester polyols.

Polyethers modified by vinyl polymers are also suitable. Products of this kind may be obtained by polymerizing, e.g. styrene and acrylonitrile in the presence of polyethers (U.S. Pat. Nos. 3,383,351; 3,304,273; 3,523,095; 3,110,695 and German Pat. No. 1,152,536).

Among the polythioethers which should be particularly mentioned are the condensation products obtained from thiodiglycol on its own and/or with other glycols, dicarboxylic acids, formaldehyde, aminocarboxylic acids or amino alcohols. The products obtained are either polythio-mixed ethers, polythioether esters or polythioether ester amides, depending on the co-components.

Suitable polyacetals include the compounds which can be prepared from aldehydes, e.g. formaldehyde, and glycols such as diethylene glycol, triethylene glycol, ethoxylated 4,4'-dihydroxy-diphenyldimethylmethane, and hexanediol-(1,6). Polyacetals suitable may also be prepared by the polymerization of cyclic acetals.

Suitable polyhydroxy polyester amides and polyamines are, for example, the predominantly linear condensates obtained from polybasic saturated and unsaturated carboxylic acids or their anhydrides and polyvalent saturated or unsaturated aminoalcohols, diamines, polyamines, and mixtures thereof.

Suitable monomers for producing hydroxyfunctional polyacrylates include acrylic acid, methacrylic acid, crotonic acid, maleic anhydride, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, glycidyl acrylate, glycidyl methacrylate, 2-isocyanatoethyl acrylate and 2-isocyanatoethyl methacrylate.

Suitable polyalkadienes include polybutadienes and polyisoprenes, such as POLY bd^{®} resin from Elf Atochem North America, Philadelphia, PA. Also included are hydrogenated polyisoprene and hydrogenated polybutadiene. Examples of those include KRATON L-2203 from Shell chemical, Houston, Tex., and POLYTAIL resins from Mitsubishi Chemical, Tokyo, Japan.

Mixtures of the above-described dihydroxy compounds can also be used.

In an embodiment, the low molecular weight diol is selected from the group consisting of ethylene glycol, diethylene glycol, propane 1,2-diol, propane 1,3-diol, butane 1,4-diol, butylene 1,3-glycol, cyclohexane diol, 1,4-cyclohexane dimethanol, hexane 1,6-diol, bisphenol A (2,2-bis(4-hydroxyphenyl)propane), hydrogenated bisphenol A (2,2-bis(4-hydroxycyclohexyl)propane), and mixtures thereof.

Suitable polyisocyanates for providing the hydrocarbon radical R₂ include organic diisocyanates having a molecular weight of from about 112 to about 1,000, and preferably from about 140 to about 400. Preferred diisocyanates are those represented by the general formula R₂(NCO)₂ in which R₂ represents a divalent aliphatic hydrocarbon group having from 4 to 18 carbon atoms, a divalent cycloaliphatic hydrocarbon group having from 5 to 15 carbon atoms, a divalent araliphatic hydrocarbon group having from 7 to 15 carbon atoms or a divalent aromatic hydrocarbon group having 6 to 15 carbon atoms. Examples of the organic diisocyanates which are suitable include tetramethylene diisocyanate, 1,6-hexamethylene diisocyanate, dodecamethylene diisocyanate, cyclohexane-1,3-and -1,4-diisocyanate, 1-isocyanato-3-isocyanatomethyl-3,5,5-trimethylcyclohexane (isophorone diisocyanate or IPDI), bis-(4-isocyanatocyclohexyl)-methane, 1,3- and 1,4-bis(isocyanatomethyl)-cyclohexane, bis-(4-isocyanato-3-methyl-cyclohexyl)-methane, isomers of toluene diisocyanate (TDI) such as 2,4-diisocyanatotoluene, 2,6-diisocyanatotoluene, mixtures of these isomers, hydrogenated TDI, 4,4'-diisocyanato diphenyl methane and its isomeric mixtures with 2,4'- and optionally 2,2'-diisocyanato diphenylmethane, and 1,5-diisocyanato naphthalene. Mixtures of diisocyanates can be used. Preferred diisocyanates are aliphatic and cycloaliphatic diisocyanates. Particularly preferred are 1,6-hexamethylene diisocyanate and isophorone diisocyanate.

In an embodiment, the polyisocyanate is selected from the group consisting of tetramethylene diisocyanate, 1,6-hexamethylene diisocyanate, dodecamethylene diisocyanate, 1,4-diisocyanatocyclohexane, 3-isocyanatomethyl-3,5,5-trimethylcyclohexylisocyanate (isophorone diisocyanate), 4,4'-diisocyanatodicyclohexylmethane, 4,4'-diisocyanatodicyclohexylpropane-(2,2), and mixtures thereof.

The prepolymer PP may be chain extended using two classes of chain extenders. The first chain extender may be compounds having the formula: H₂N-R₄-NH₂; wherein R₄ represents an alkylene or alkylene oxide radical not substituted with ionic or potentially ionic groups. Alkylene diamines include hydrazine, ethylenediamine, propylenediamine, 1,4-butylenediamine and piperazine. The alkylene oxide diamines include dipropylamine diethyleneglycol (DPA-DEG available from Tomah Products, Milton, WI), 2-methyl-1,5-pentanediamine (Dytec A from DuPont), hexane diamine, isophorone diamine, and 4,4-methylenedi(cyclohexylamine), and the DPA-series ether amines available from Tomah Products, Milton, WI, including dipropylamine propyleneglycol, dipropylamine dipropyleneglycol, dipropylamine tripropyleneglycol, dipropylamine poly(propylene glycol), dipropylamine ethyleneglycol, dipropylamine poly(ethylene glycol), dipropylamine 1,3-propane diol, dipropylamine 2-methyl-1,3-propane diol, dipropylamine 1,4-butane diol, dipropylamine 1,3-butane diol, dipropylamine 1,6-hexane diol and dipropylamine cyclohexane-1,4-dimethanol. Mixtures of the listed diamines may also be used.

In an embodiment, the first chain extender is a mixture of dipropylamine-diethyleneglycol and diethyleneamine.

The second chain extender may be compounds having the formula: H₂N-R₅-NH₂, wherein R₅ represents an alkylene radical substituted with ionic or potentially ionic groups. Such compounds have an ionic or potentially ionic group and two groups that are reactive with isocyanate groups. Such compounds contain two isocyanate-reactive groups and an ionic group or group capable of forming an ionic group. The ionic group or potentially ionic group can be selected from the group consisting of ternary or quaternary ammonium groups, groups convertible into such a group, a carboxyl group, a carboxylate group, a sulfonic acid group and a sulfonate group. The at least partial conversion of the groups convertible into salt groups of the type mentioned may take place before or during the mixing with water. Specific compounds include diaminosulfonates, such as for example the sodium salt ofN-(2-aminoethyl)-2-aminoethane sulfonic acid (AAS) or the sodium salt of N-(2-aminoethyl)-2- aminopropionic acid.

In an embodiment, the second chain extender is the sodium salt of N-(2-aminoethyl)-2-aminoethane sulfonic acid.

The polyurethane polymer may also include compounds which are situated in each case at the chain ends and terminate said chains (chain terminators). These chain terminators can be derived from compounds having the Formula CT: 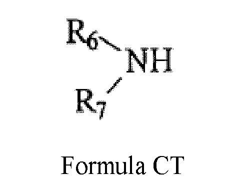 wherein R₆ is an H atom or alkylene radical optionally having a hydroxyl end and R₇ is alkylene radical optionally having a hydroxyl end. Suitable compounds include compounds such as monoamines, particularly monosecondary amines, or monoalcohols. Examples include: methylamine, ethylamine, propylamine, butylamine, octylamine, laurylamine, stearylamine, isononyloxy-propylamine, dimethylamine, diethylamine, dipropylamine, dibutylamine, N-methylaminopropylamine, diethyl(methyl)aminopropylamine, morpholine, piperidine, diethanolamine and suitable substituted derivatives thereof, amide amines of diprimary amines and monocarboxylic acids, monoketimes of diprimary amines, primary/tertiary amines such as N,N-dimethylamino-propylamine and the like. Also suitable are chain terminating alcohols such as, C1 to C10 or higher alcohols including, methanol, butanol, hexanol, 2-ethylhexyl alcohol, isodecyl alcohol, and the like and even mixtures thereof, as well as amino-alcohols such as aminomethylpropanol (AMP).

In an embodiment, the chain terminator is selected from the group consisting of methylamine, ethylamine, propylamine, butylamine, octylamine, laurylamine, stearylamine, isononyloxy-propylamine, dimethylamine, diethylamine, dipropylamine, dibutylamine, N-methylaminopropylamine, diethyl(methyl)amino-propylamine, morpholine, piperidine and diethanolamine, amide amines of diprimary amines and monocarboxylic acids, monoketimes of diprimary amines, primary/tertiary amines, methanol, butanol, hexanol, 2-ethylhexyl alcohol, isodecyl alcohol, aminomethylpropanol, and mixtures thereof.

In an embodiment, diethylene glycol is incorporated into the polyurethane dispersion either as the low molecular weight diol, or as part of the non-ionic chain extender through the use of dipropylamine-diethyleneglycol. If the diethylene glycol is used as the low molecular weight diol, then preferably the DPA-DEG is not used as the non-ionic chain extender. Likewise, if the DPA-DEG is used as the non-ionic chain extender, then diethylene glycol is preferably not used as the low molecular weight diol. The use of the diethylene glycol or DPA-DEG is especially desirable when the polyurethane dispersion is incorporated into a hair fixative, as the diethylene glycol significantly increases the adhesion to hair.

In an embodiment, n is from 1 to 3, and m is from 1 to 5.

A preferred embodiment relates to a composition comprising a dispersed polyurethane comprising a polymer P^{x} which is the reaction products of:
A) a prepolymer according to the Formula PP, as shown above;
   wherein
   R₁ represents a bivalent radical of a dihydroxyl functional compound,
   R₂ represents a hydrocarbon radical of an aliphatic or cycloaliphatic polyisocyanate,
   R₃ represents a radical of a low molecular weight diol, optionally substituted with ionic groups,
   n is from 0 to 5, optionally 1 to 3, and m is >1, optionally 1 to 5;
B) at least one first chain extender being a mixture of dipropylamine-diethyleneglycol and diethyleneamine; and
C) at least one second chain extender being the sodium salt of N-(2-aminoethyl)-2-aminoethane sulfonic acid;
   and wherein the reactants further include a chain terminator according to Formula CT, as shown above;
   wherein R₆ is an H atom or alkylene radical optionally having a hydroxyl end and R₇ is alkylene radical optionally having a hydroxyl end;
   wherein at least one of R₁, R₂ and R₃ comprises a group according to Formula 3, as shown above; wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

In an embodiment, polymer P or polymer P^{x} is a polyurethane, preferably an anionic polyurethane. In an embodiment, the sole polymer P or sole polymer P^{x} is a polyurethane, preferably an anionic polyurethane. In an embodiment, polymer P or polymer P^{x} is a polyurethane formed by reacting a copolymer of hexanediol, neopentyl glycol (2,2-dimethyl-1,3-propanediol), and adipic acid (1,6-hexanedioc acid) with hexamethylene diisocyanate, and then further reacting the resulting polymer with N-(2-aminoethyl)-3-aminoethanesulfonic acid and ethylenediamine. Polymer P may have the INCI name Polyurethane-34. In an embodiment, polymer P or polymer P^{x} is an anionic polyurethane in the form of an aqueous dispersion.

An example of a dispersed polyurethane is discussed in EP1970391A2, the applicant of which is Bayer MaterialScience LLC. Suitable commercially available dispersed polyurethanes include Baycusan^{®} C 1001 from Bayer Material Science.

The viscosity of the composition and of the formulation comprising polymer P and/or polymer P^{x} may be from about 1 to about 30000 mPa·s, preferably from about 1 to about 15000 mPa·s, more preferably from about 1 to about 7500 mPa·s, even more preferably from about 1 to about 4000 mPa·s, measured at 23°C according to DIN EN ISO 3219. In an embodiment, the viscosity is from about 1000 to about 2000 mPa·s, measured at 23°C according to DIN EN ISO 3219.

In an embodiment, the pH composition or formulation comprising polymer P and/or polymer P^{x} may be less about pH 7, alternatively less than about pH 6, alternatively less than about pH 5, alternatively less than about pH 4.

An embodiment relates to a composition, wherein the direct crosslink is an intra-strand crosslink and thus is a direct covalent bond between the Cα of the polymer P^{x} and a second C^{α} of the same polymer P^{x} molecule, and wherein said direct crosslink comprises no intermediary atom. Another embodiment relates to a composition, wherein the direct crosslink is an inter-strand crosslink and thus the direct crosslink is a direct covalent bond between the Cα of the polymer P^{x} and a second C^{α} of a different polymer P^{x} molecule, and wherein said direct crosslink comprises no intermediary atom. In another embodiment, mixtures of inter-strand and intra-strand direct crosslinks are present, wherein the direct crosslink is a direct covalent bond between two Cα atoms, and wherein said direct crosslink comprises no intermediary atom.

An embodiment relates to a composition, wherein Rⁱ comprises an electron-withdrawing functional group selected from the group consisting of: carboxyl; thiocarboxyl; carbonyl; thiocarbonyl; imine; hydroxyl; aryl; heteroaryl; a saturated cyclic radical; alkoxy; amino; amide; imide; phosphorus; sulphur; tin; silica; and alkyne. Alternatively the electron-withdrawing functional group is selected from the group consisting of: carboxyl; thiocarboxyl; carbonyl; thiocarbonyl; imine; hydroxyl; aryl; heteroaryl; a saturated cyclic radical; alkoxy; amino; amide; imide; and alkyne.

An embodiment relates to cosmetic composition comprising:
(a) the composition, as described here; and
(b) a cosmetically acceptable carrier.

An embodiment relates to a composition, as described herein, further comprising a cosmetically acceptable carrier.

In an embodiment of the cosmetic composition, the cosmetically acceptable carrier is water and the composition comprises from about 0.001% to about 10%, preferably from about 0.1% to about 9.0%, more preferably from about 2.0% to about 8.0%, even more preferably from about 3% to about 7.0%, most preferably from about 4.0% to about 6.0% of polymer P^{x}, by total weight of the cosmetic composition.

In an embodiment of the cosmetic composition, the cosmetic composition is a hairstyling composition. In an embodiment, the hairstyling composition is for imparting hold and comprises from about 3% to about 10% preferably from about 3.5% to about 9.5%, more preferably from about 4.0% to about 9.0%, even more preferably from about 4% to about 8.0%, most preferably from about 5.0% to about 7.0% of polymer P^{x}, by total weight of the hairstyling composition. In another embodiment, the hairstyling composition is for imparting volume and comprises from about 0.001% to about 3%, preferably from about 0.01% to about 2.5%, more preferably from about 0.1% to about 2.3%, even more preferably from about 1% to about 2.2%, most preferably from about 1.5% to about 2.0% of polymer P^{x}, by total weight of the hairstyling composition.

In an embodiment, the polymer P^{x} is substantially free of indirect crosslinks. "Substantially free of indirect crosslinks" means that less than about 2%, preferably less than about 1.5%, more preferably less than about 1%, even more preferably less than about 0.5%, most preferably less than about 0.001%, optimally about 0% of the total crosslinks (includes direct and indirect) are indirect crosslinks.

In an embodiment, a composition or formulation comprising polymer P^{x} and/or polymer P is substantially free of a crosslinking agent or a derivative thereof. "Substantially free of a crosslinking agent" means that the composition and/or formulation comprises less than about 2%, preferably less than about 1.5%, more preferably less than about 1%, even more preferably less than about 0.5%, most preferably less than about 0.001%, optimally about 0% by weight of at least one crosslinking agent, by total weight of the composition or formulation. The crosslinking agent may be selected from the group consisting of: methylene-*bis*-acrylamide; di(meth)acrylate; *N*-(1-hydroxy-2,2-dimethoxyethyl)acrylamide; ethyleneglycol dimethacrylate; ethyleneglycol diacrylate; allylmethacrylate; 1,1,1-trimethylolpropane triacrylate; triallylamine; tetraallyloxethane; and mixtures thereof. The second method is carried out after derivatisation of reactive groups in the polymer. The crosslinking agent may be capable of forming an indirect crosslink selected from the group consisting of: ester bond; amide bond; ether bond. In an embodiment, the crosslinking agent is a molecule comprising at least three atoms. In an embodiment, the crosslinking agent is selected from the group consisting of bifuntional and trifunctional crosslinking agents. In an embodiment, independently, a formulation, composition, kit, process, use, and/or method pursuant to the present invention and described herein is substantially free of a crosslinking agent.

The photoinitiator may be a persulfate salt. The persulfate salt may be selected from the group consisting of: sodium persulfate; potassium persulfate; ammonium persulfate; lithium persulfate; and mixtures thereof. A persulfate salt is preferred because they are easy to handle e.g. stable under dry conditions, highly water soluble, cheap, and leaves no toxicologically-relevant products (it leaves a hydrogensulfate as reaction product), and forms, with each homolytic cleavage, two equal radicals at the same time. In an embodiment, the persulfate salt is selected from the group consisting of: ammonium peroxodisulfate; sodium peroxodisulfate; potassium peroxodisulfate; lithium peroxodisulfate; and mixtures thereof. The photoinitiator may be in the form of a powder. An embodiment comprises ammonium peroxodisulfate as sole photoinitiator.

In an embodiment, the composition, according to any embodiment above, further comprises a reaction product of a photoinitiator. The reaction product of a photoinitiator may be selected from the group consisting of: ammonium hydrogensulfate; sodium hydrogensulfate; potassium hydrogensulfate; lithium hydrogensulfate; and mixtures thereof. An embodiment comprises ammonium hydrogensulfate as sole reaction product of a photoinitiator.

The weight ratio of photoinitiator to the polymer P (i.e. photoinitiator : polymer P) is from about 1:100 to about 1:1, preferably from about 2:100 to about 50:100, more preferably from about 5:100 to about 30:100, most preferably from about 10:100 to about 20:100.

The irradiation and/or the activation of the photoinitiator may be achieved upon exposure to UV at a wavelength of from about 170 nm to about 400 nm, preferably from about 190 nm to about 300 nm, more preferably from about 200 nm to about 280 nm, even more preferably from about 200 nm to about 260 nm.

The UV may be provided by a UV source, for example a UV lamp. Suitable UV lamps are those made by Heraeus Holding GmbH, Germany, particularly: a medium pressure mercury lamp (TQ 150) with power consumption of 150 Watt and an effective spectral range between 200 nm and 300 nm; and a low pressure lamp, ozone free, output peak at 254 nm, 30 Watt. Light emitting diodes (LEDs), wherein the emitted light is UV, may also be employed.

The duration of the irradiation may be from about 0.001 sec to about 22000 sec, preferably from about 10 sec to about 10000 sec, more preferably from about from about 20 sec to about 6000 sec, most preferably from about 60 sec to about 3600 sec. In an embodiment, the duration of the irradiation is from about 5 min to about 20 min.

The irradiation may be carried out under agitation. It is important that the liquid to be irradiated (e.g. a composition or formulation as disclosure herein) is provided to the UV source e.g. UV lamp, with a relatively high surface area under agitation such that an even and wide distribution of photoinitiator molecules are activated, so that a wide distribution of crosslinks are formed in the polymer and that not all crosslinks occur in a concentrated area, for example. This is particularly important if the liquid to be irradiated is not transparent e.g. milky or translucent liquids. A form of agitation is stirring, which may be carried out by a stirring means. The stirring means may be a common device capable of stirring liquid cosmetic compositions and formulations. The following types of stirring means may be utilised: magnetic stirring device and flea; stirring rod with 'drill-type' stirring adaptor. Suitable stirring means are made by the manufacturer IKO^{®} Werke GmbH & Co. KG in Germany. Stirring may be carried out at the fastest speed possible such that the liquid does not splash. Typically a medium stirring setting is employed.

Another means for agitation is pouring or flowing the liquid to be irradiated past the UV source. This may occur via the use of tubing, or a shaking device such as a tilting table or shaker. Another method of ensuring that there is sufficient surface area is to provide the liquid to be irradiated in a receptacle, the surface of which is coated in a thin film of the liquid to be irradiated.

Safe simultaneous irradiation and stirring may, for example, be carried out by placing the UV lamp in an opaque box e.g. made from wood, UV-resistant plastic or metal, placing the receptacle, which comprises the flea and the liquid to be irradiated, under the lamp, and placing the box on top of the magnetic stirrer. An alternative method of safe simultaneous irradiation and stirring may be the use of a UV source typically used for crosslinking nucleic acid to nitrocellulose membranes in Southern blotting e.g. a Stratalinker^{®}, from Stratagene, which delivers UV at 254 nm. The liquid to be irradiated is placed in a receptable such that a high surface of the liquid to be irradiated is available to the UV source. The receptacle is then placed in the Stratalinker^{®}. The Stratalinker^{®} may be placed in a shaking incubator, set a room temperature and an appropriate shaking level such that the liquid to be irradiated does not splash.

The activation of the photoinitiator and the formation of the direct crosslink may be concomitant. Alternatively, the activation of the photoinitiator may be conducted prior forming the direct crosslink. Activating the photoinitiator prior forming the direct crosslink is preferred as it may prevent the polymer from being damaged by the irradiation.

From about 0.01% to about 100%, preferably from about 0.1% to about 50%, more preferably from about 1% to about 5%, of alpha-carbons of polymer P^{x} may be crosslinked with a plurality of direct crosslinks. When only proportion of the alpha-carbons are directly crosslinked, the polymer P^{x} comprises both alpha-carbons in a crosslinked form and alpha-carbons in an uncrosslinked form. The degree of elasticity varies depending on the degree of direct crosslinking, i.e. the proportion of alpha-carbons being directly crosslinked.

In an embodiment, polymer P^{x} comprises a mixture of indirect crosslinks being labile peroxo-bridges and direct crosslinks being carbon-carbon bonds, wherein the labile peroxo-bridges are the sole indirect crosslinks present in polymer P^{x}.

The polymer P and/or polymer P^{x} may be substantially free of acrylate groups.

The composition and/or formulation of the present invention may comprise a cationic surfactant. Non-limiting examples of such cationic surfactants are described in the CTFA Cosmetic Ingredient Dictionary, 3rd edition, edited by Estrin, Crosley, and Haynes, (The Cosmetic, Toiletry, and Fragrance Association, Inc., Washington, D.C. (1982)).

Suitable cationic surfactants include monoalkyl quaternized ammonium salt cationic surfactants useful herein are those having one alkyl chain which has from 12 to 22 carbon atoms, preferably from 16 to 22 carbon atoms, from 16 to 22 carbon atoms, even more preferably a C18-22 alkyl group. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms. Such mono-alkyl cationic surfactants include, for example, mono-alkyl quaternary ammonium salts and mono-alkyl amines. Mono-alkyl quaternary ammonium salts include, for example, those having a non-functionalized alkyl chain and those having a functionalized long alkyl chain such as those having an ester-linkage. Mono-alkyl amines include, for example, mono-alkyl amidoamines and salts thereof.

It is believed that monoalkyl cationic surfactants having a longer alkyl group provide improved deposition on the hair, thus can provide improved conditioning benefits such as improved softness on dry hair, compared to cationic surfactant having a shorter alkyl group. It is also believed that such cationic surfactants can provide reduced irritation, compared to cationic surfactants having a shorter alkyl group.

Suitable cationic surfactants include mono-long alkyl quaternized ammonium salts having the Formula ML 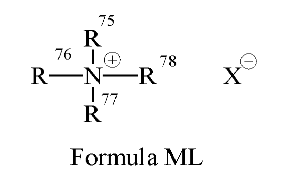 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to 30 carbon atoms; and wherein R⁷⁵, R⁷⁶, R⁷⁷ and R⁷⁸ are independently selected from an alkyl group of from 1 to 4 carbon atoms or an alkoxy, polyoxyalkylene, alkylamido, hydroxyalkyl, aryl or alkylaryl group having up to 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g. those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 22 carbon atoms, still more preferably from 18 to 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Examples of mono-long alkyl quaternized ammonium salt cationic surfactants include: behenyltrimethyl ammonium salt; stearyltrimethyl ammonium salt; cetyltrimethyl ammonium salt; and hydrogenated tallow alkyltrimethyl ammonium salt.

Mono-alkyl amines are also suitable as cationic surfactants. Primary, secondary, and tertiary fatty amines are useful. Particularly useful are tertiary amido amines having an alkyl group of from 12 to 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al. These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from about 1:0.3 to about 1:2, more preferably from about 1:0.4 to about 1:1.

Other cationic surfactants such as di-alkyl chain cationic surfactants may also be used alone, or in combination with mono-alkyl chain cationic surfactants. Such di-alkyl chain cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride. Such di-alkyl chain cationic surfactants also include, for example, tallowalkyl (2-ethylhexyl) dimethyl ammonium methosulfate which is available, for example, from Akzo Nobel with a tradename Arquad HTL8-MS.

Non-limiting examples of particularly preferred cationic surfactants include those conforming to Formula CA: 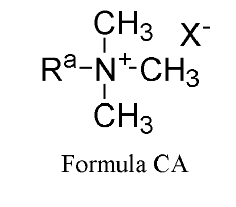 wherein R^{a} is an aliphatic moiety comprising from 12 carbon atoms to 22 carbon atoms, preferably from 16 to 22 carbon atoms. In an embodiment R^{a} is a saturated aliphatic moiety comprising from 16 carbon atoms to 22 carbon atoms. In an embodiment, the cationic surfactant is selected from the group consisting of cetyltrimethyl ammonium salt, stearyl trimethylammonium salt, behenyltrimethyl ammonium salt, and mixtures thereof. The most preferred cationic polymer is cetyltrimethyl ammonium chloride.

The cationic surfactant may be present in a composition or formulation, as described herein, in an amount of from about 0.001% to about 10%, preferably from 0.01% to about 5.0%, more preferably from about 0.05% to about 2.0%, most preferably from about 0.1% to about 1.0%, by total weight of the composition or formulation.

In an embodiment of the hairstyling composition for imparting volume, the composition further comprises a cationic surfactant. In another embodiment, hairstyling composition for imparting volume, the cationic surfactant is present in amount of from about 0.001% to about 10%, preferably from 0.01% to about 5.0%, more preferably from about 0.05% to about 2.0%, most preferably from about 0.1% to about 1.0%, by total weight of the hairstyling composition for imparting volume.

Other conditioning agents, in particular silicones, may be included in a composition/formulation as detailed herein. Conditioning agents include any material which is used to give a particular conditioning benefit to hair and/or skin. For hair treatment compositions, suitable conditioning agents are those which deliver one or more benefits relating to shine, softness, combability, antistatic properties, wet-handling, damage, manageability, body, and greasiness. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof. Such conditioning agents should be physically and chemically compatible with the essential components of the composition, and should not otherwise unduly impair product stability, aesthetics or performance.

The concentration of the conditioning agent in the composition should be sufficient to provide the desired conditioning benefits, and as will be apparent to one of ordinary skill in the art. Such concentration can vary with the conditioning agent, the conditioning performance desired, the type and concentration of other components, and other like factors.

The viscosity-increasing agent may be selected from the group consisting of non-ionic thickeners, cationic thickeners, anionic thickeners, amphoteric thickeners, and mixtures thereof. The viscosity-increasing agent may be present in the composition in an amount of from about 0.1% to about 10%, preferably about 0.2% to about 5.0%, by total weight of the composition. Non-ionic viscosity increasing agents are preferred due to the reduced risk of causing residues or build up on the hair. In an embodiment, the viscosity-increasing agent is a non-ionic thickener. In an embodiment the viscosity-increasing agent is a polysaccharide polymer, preferably a cellulose-based polymer, more preferably hydroxyethylcellulose.

A composition or formulation as described herein, or a plurality thereof, may comprise a cosmetically acceptable carrier. The composition or formulation, for example the monomer composition or oxidising formulation, may comprise from about 60% to about 99.9%, alternatively from about 70% to about 95%, and alternatively from about 80% to about 90%, of a cosmetically acceptable carrier, by total weight of the composition or formulation. Cosmetically acceptable carriers suitable for use include, for example, those used in the formulation of tonics and gels. Cosmetically acceptable carrier may comprise water; silicones such as volatile silicones, amino or non-amino silicone gums; organic compounds such as C₂-C₁₀ alkanes, acetone, methyl ethyl ketone, volatile organic C₁-C₁₂ alcohols, esters of C₁-C₂₀ acids and of C₁-C₈ alcohols such as methyl acetate, butyl acetate, ethyl acetate, and isopropyl myristate, dimethoxyethane, diethoxyethane, C₁₀-C₃₀ fatty alcohols such as lauryl alcohol, cetyl alcohol, stearyl alcohol, and behenyl alcohol; C₁₀-C₃₀ fatty acids such as lauric acid and stearic acid; C₁₀-C₃₀ fatty amides such as lauric diethanolamide; C₁₀-C₃₀ fatty alkyl esters such as C₁₀-C₃₀ fatty alkyl benzoates; hydroxypropylcellulose, and mixtures thereof. In an embodiment, the carrier comprises water, fatty alcohols, volatile organic alcohols, and mixtures thereof. In a most preferred embodiment, the carrier is water. The composition and/or formulation may comprise an alcohol in order to increase the drying time on hair. In an embodiment, the composition and/or formulation may comprises from about 1% to about 20% alcohol, alternatively 10% to 18% alcohol, by total weight of the composition or formulation, wherein the alcohol is preferably a C₁-C₁₂ alcohol. The composition and/or formulation may comprise a non-ionic surfactant.

A composition or formulation as described herein may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizers, surfactants (which may be anionic, cationic, amphoteric or nonionic), neutralizing agents, propellants, hair conditioning agents (e.g., silicone fluids, fatty esters, fatty alcohols, long chain hydrocarbons, cationic surfactants, etc.), emollients, lubricants and penetrants such as various lanolin compounds, vitamins, proteins, preservatives, dyes, tints, bleaches, reducing agents and other colorants, sunscreens, gelling agents, physiologically active compounds for treating the hair or skin (e.g., anti-dandruff actives, hair growth actives), non-polymeric thickeners including clays, and perfume.

A composition or formulation as described herein may be in different product forms. It may be in a form selected from the group consisting of a gel, an emulsion, a cream, a spray, a lotion, or a mousse.

According to another aspect, the present invention relates to a method of styling hair comprising:
(a) providing a composition, as detailed herein;
(b) applying said composition to hair;
(c) styling the hair.

In another embodiment, the method comprises:
(a) styling the hair;
(b) providing a composition, as detailed herein, and applying said composition to hair.

According to another aspect, the present invention relates to the use of a composition, as detailed herein, for styling hair. In another embodiment, the styling hair is selected from the group consisting of: imparting volume, hold and/or body to hair; retaining curls; improving combability; and mixtures thereof.

According to another aspect, the present invention relates to a kit comprising:
(a) a cosmetic formulation comprising:
   i. a polymer P comprising a moiety of formula selected from the group consisting of: Formula 1, Formula 2, and a combination of Formula 1 and Formula 2, as shown above;
      wherein R^{x} comprises a group according to Formula 3, as shown above; and wherein R^{y} and R^{z} are, independently, any suitable atom or moiety; and Rⁱ is an electron-withdrawing functional group;
   ii. a cosmetically acceptable carrier;
(b) a photoinitiator;
   wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

The kit may be substantially free from a crosslinking agent, optionally a crosslinking agent as detailed herein.

In another embodiment of the kit, the polymer P comprises a moiety of formula selected from the group consisting of Formula 4 and Formula 5, as shown above;
wherein R^{x} comprises a group according to Formula 3, as shown above;
wherein R^{y} and R^{z} are, independently, any suitable atom or moiety; and Rⁱ is an electron-withdrawing functional group; and wherein f is from 4 to 40;
wherein polymer P is capable of forming at least one direct crosslink, wherein the direct crosslink is a direct covalent bond between the C^{α} of the polymer P and a second C^{α} of a polymer P, and wherein said direct crosslink comprises no intermediary atom.

Alternatively, the polymer P comprises a moiety of formula selected from the group consisting of Formula 4, as shown above;
wherein R^{x} comprises a group according to Formula 3, as shown above;
wherein R^{y} and R^{z} are, independently, any suitable atom or moiety; and Rⁱ is an electron-withdrawing functional group; and wherein f is from 2 to 40, or 4 to 40, or from 5 to 40, or from 10 to 40;
wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

In another embodiment of the kit, the polymer P is the reaction products of:
A) a prepolymer according to the Formula PP, as shown above;
   wherein
   R₁ represents a bivalent radical of a dihydroxyl functional compound,
   R₂ represents a hydrocarbon radical of an aliphatic or cycloaliphatic polyisocyanate,
   R₃ represents a radical of a low molecular weight diol, optionally substituted with ionic groups,
   n is from 0 to 5, and m is >1;
B) at least one first chain extender being a mixture of dipropylamine-diethyleneglycol and diethyleneamine.; and
C) at least one second chain extender being the sodium salt of N-(2-aminoethyl)-2-aminoethane sulfonic acid;
and wherein the reactants further include a chain terminator according to the Formula CT, as shown above;
wherein R₆ is an H atom or alkylene radical optionally having a hydroxyl end and R₇ is alkylene radical optionally having a hydroxyl end;
wherein at least one of R₁, R₂ and R₃ comprises a group according to Formula 3, as shown above; wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

In a preferred embodiment of the kit, the cosmetic formulation comprises a dispersed polyurethane comprising a polymer P which is the reaction products of:
A) a prepolymer according to the Formula PP, as shown above;
   wherein
   R₁ represents a bivalent radical of a dihydroxyl functional compound,
   R₂ represents a hydrocarbon radical of an aliphatic or cycloaliphatic polyisocyanate,
   R₃ represents a radical of a low molecular weight diol, optionally substituted with ionic groups,
   n is from 1 to 3, and m is from 1 to 5;
B) at least one first chain extender being a mixture of dipropylamine-diethyleneglycol and diethyleneamine; and
C) at least one second chain extender being the sodium salt of N-(2-aminoethyl)-2-aminoethane sulfonic acid;
and wherein the reactants further include a chain terminator according to Formula CT, as shown above;
wherein R₆ is an H atom or alkylene radical optionally having a hydroxyl end and R₇ is alkylene radical optionally having a hydroxyl end;
wherein at least one of R₁, R₂ and R₃ comprises a group according to Formula 3, as shown above; wherein polymer P is capable of forming at least one direct crosslink, preferably a plurality of direct crosslinks, wherein the direct crosslink is a direct covalent bond between the Cα of the polymer P and a second C^{α} of a polymer P, and wherein said direct crosslink comprises no intermediary atom.

The kit may further comprise:
(c) a device for irradiating the cosmetic formulation with UV;
(d) instructions.

The kit may further comprise a stirring means, preferably a stirring means as detailed herein.

The photoinitiator may be a photoinitiator as detailed herein. In a preferred embodiment, the photoinitiator is a persulfate salt, preferably ammonium peroxodisulfate.

In an embodiment, the cosmetic formulation is a hairstyling formulation. In an embodiment, the hairstyling formulation is for imparting hold and comprises from about 3% to about 10% preferably from about 3.5% to about 9.5%, more preferably from about 4.0% to about 9.0%, even more preferably from about 4% to about 8.0%, most preferably from about 5.0% to about 7.0% of polymer P^{x}, by total weight of the hairstyling formulation. In another embodiment, the hairstyling formulation is for imparting volume and comprises from about 0.001% to about 3%, preferably from about 0.01% to about 2.5%, more preferably from about 0.1% to about 2.3%, even more preferably from about 1% to about 2.2%, most preferably from about 1.5% to about 2.0% of polymer P^{x}, by total weight of the hairstyling formulation.

According to another aspect, the present invention relates to a method of styling hair comprising:
I. providing the kit, as detailed herein;
II. mixing the cosmetic formulation with the photoinitiator resulting in a mixed cosmetic formulation;
III. irradiating said mixed cosmetic formulation with UV resulting in an irradiated cosmetic formulation;
IV. applying the irradiated cosmetic formulation to hair;
V. styling the hair;
VI. exposing the hair to a temperature of from about 20°C to about 80°C, preferably from about 30°C to about 60°C, more preferably from about 35°C to about 55°C, even more preferably from about 40°C to about 50°C.

The mixing may be carried out by agitation as detailed above. In an embodiment, the photoinitiator is provided as a powder. Mixing is complete when the powder is dissolved in the cosmetic formulation. The irradiating may be carried out via the use of a UV source, as detailed above. The exposing the hair to a temperature may be carried out by utilisation of a hood device, blow dryer, and/or flat iron.

The method may further comprise providing and applying to hair a hair care and/or hair styling active. Additionally or alternatively it may comprise providing and utilising an implement for applying styling effects to the hair. Said hair care and/or hair styling active may be selected from the group consisting of hair fixing polymers; conditioning agents, particularly cationic polymers; cleansing agents, particularly surfactants; thickeners; glossing agents; shine-imparting agents; dyes or colour-imparting agents; glitter or coloured particles; silicones; and mixtures thereof. The hair care and/or styling active may be present in a composition selected from the group consisting of waxes; gels; hair sprays; mousses; conditioning compositions, particularly leave-in conditioning compositions; finishing sprays; glossing or shine-imparting products; and mixtures thereof. The implement may be selected from the group consisting of blow dryers; flat irons, combs, brushes, curlers, curling tongs, electrical curling devices, foils, scissors, clips, hair bands, and mixtures thereof.

According to another aspect, the present invention relates to a process comprising:
(a) the provision of a formulation comprising a polymer P, wherein polymer P comprises a moiety of formula selected from the group consisting of: Formula 1, Formula 2, and a combination of Formula 1 and Formula 2, as shown above; wherein R^{x} comprises a group according to Formula 3, as shown above;
   and wherein R^{y} and R^{z} are, independently, any suitable atom or moiety;
   and Rⁱ is an electron-withdrawing functional group;
(b) the provision of a photoinitiator;
(c) mixing said polymer P with said photoinitiator to form a mixed formulation;
(d) the irradiation of said mixed formulation with UV to form at least one direct crosslink, wherein the direct crosslink is a direct covalent bond between the C^{α} of the polymer P and a second C^{α} of a polymer P, and wherein said direct crosslink comprises no intermediary atom.

The process may be substantially free from the use and/or application of a crosslinking agent, optionally a crosslinking agent as detailed herein.

In an embodiment of the process, the polymer P is the reaction products of:
A) a prepolymer according to the Formula PP, as shown above;
   wherein
   R₁ represents a bivalent radical of a dihydroxyl functional compound,
   R₂ represents a hydrocarbon radical of an aliphatic or cycloaliphatic polyisocyanate,
   R₃ represents a radical of a low molecular weight diol, optionally substituted with ionic groups,
   n is from 1 to 3, and m is from 1 to 5;
B) at least one first chain extender being a mixture of dipropylamine-diethyleneglycol and diethyleneamine; and
C) at least one second chain extender being the sodium salt of N-(2-aminoethyl)-2-aminoethane sulfonic acid;
   and wherein the reactants further include a chain terminator according to Formula CT, as shown above;
   wherein R₆ is an H atom or alkylene radical optionally having a hydroxyl end and R₇ is alkylene radical optionally having a hydroxyl end;
   wherein at least one of R₁, R₂ and R₃ comprises a group according to Formula 3, as shown above; wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

In an embodiment of the process, Rⁱ comprises an electron-withdrawing functional group selected from the group consisting of: carboxyl; thiocarboxyl; carbonyl; thiocarbonyl; imine; hydroxyl; aryl; heteroaryl; a saturated cyclic radical; alkoxy; amino; amide; imide; phosphorus; sulphur; tin; silica; and alkyne. Alternatively the electron-withdrawing functional group is selected from the group consisting of: carboxyl; thiocarboxyl; carbonyl; thiocarbonyl; imine; hydroxyl; aryl; heteroaryl; a saturated cyclic radical; alkoxy; amino; amide; imide; and alkyne.

In an embodiment of the process, the pH of the formulation decreases following irradiation with UV. In an embodiment of the process, during the irradiation, the formulation is under vigorous agitation, preferably by a stirring means, preferably a stirring means as detailed herein.

In another embodiment of the process, the formulation is a cosmetic formulation as detailed herein.

According to another aspect, the present invention relates to the use of a kit, as detailed herein, for styling hair. In another embodiment, the styling hair is selected from the group consisting of: imparting volume, hold and/or body to hair; retaining curls; improving combability; and mixtures thereof.

According to another aspect, the present invention may further relate to an article of commerce comprising at least one composition or formulation as described herein, or a plurality thereof, and a communication pertaining to the composition and/or formulation. The communication may be printed material attached directly or indirectly to packaging containing at least one composition and/or formulation pursuant to the present invention. Alternatively, the communication may be an electronic or a broadcast message that is associated with a hairstyling device and/or the composition and/or formulation. The communication may comprise images comparing the appearance of a person prior to use of the composition and/or formulation to the appearance of the same person after using the composition and/or formulation.

According to a further aspect, the present invention comprises a method of marketing a kit as described herein, wherein the method of marketing comprises the step of making available to a consumer the kit, and providing a communication to the consumer that the kit may provide one or more styling benefits to the hair.

### EXAMPLES

The following examples further describe and demonstrate the preferred embodiments within the scope of the present invention. The examples are given solely for the purpose of illustration, and are not to be construed as limitations of the present invention since many variations thereof are possible without departing from its scope.

An example hairstyling composition for imparting hold comprises:
8.0% directly crosslinked Baycusan^{®} C 1001;
1.5% PEG-12 dimethicone;
0.2% PPG-1/PEG-9 lauryl glycol ether;
0.4% hydroxyethylcellulose;
16.5% ethanol;
qsp 100% with water.

An example hairstyling composition for imparting volume comprises:
2.0% directly crosslinked Baycusan^{®} C 1001;
0.1% cetyltrimethylammonium chloride;
0.4% hydroxyethylcellulose;
16.5% ethanol;
qsp 100% with water.

### EXPERIMENTS

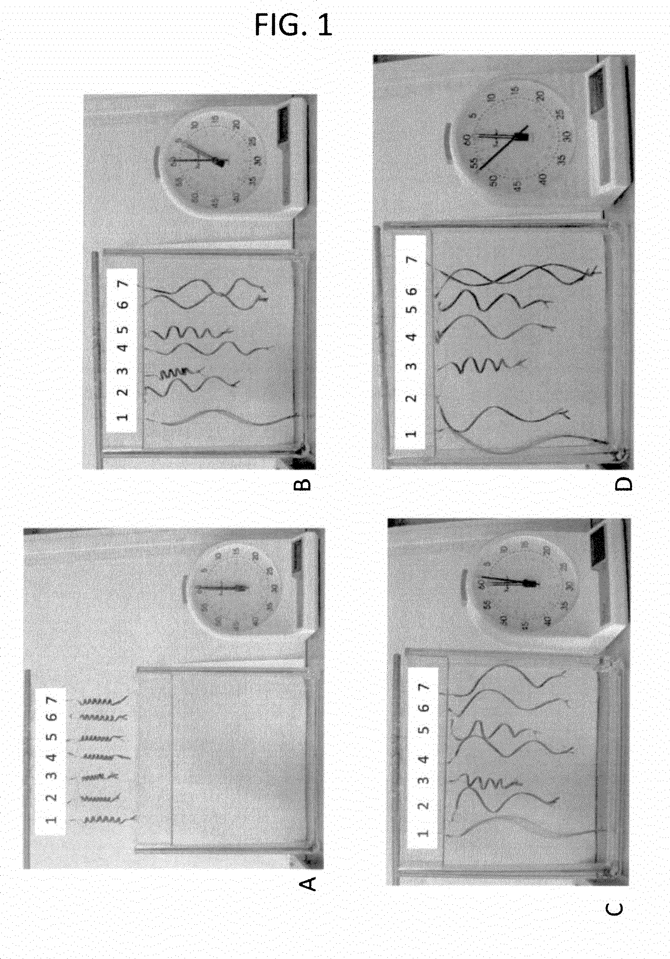

Figs. 1 to 3 demonstrate aspects of the efficacy of the present invention. For all Figs. 1 to 3, the polyurethane utilised in the experiments was Baycusan^{®} C1001 polyurethane dispersion from Bayer MaterialScience. As used below and unless stated otherwise "crosslinked polyurethane" means directly crosslinked polyurethane.
Fig. 1: Wet curl retention test - crosslinked versus uncrosslinked. Hair samples were treated, from left to right, with: (1) water only; (2) 8% of untreated polyurethane dispersion in water; (3) 8% of a polyurethane dispersion in water; (4) 4% of untreated polyurethane dispersion in water; (5) 4% of a polyurethane dispersion in water; (6) 2% of untreated polyurethane dispersion in water; (7) 2% of a polyurethane dispersion in water. Immediately following treatment, the hair samples were wound onto helical rollers, dried for one hour at room temperature or UV irradiated at room temperature on a "UV barbecue", dried at 40°C overnight, removed from the roller, a weight added to the end of the curl and then immersed in water in order to evaluate the ability of each sample to retain the curl. Samples 3, 5 and 7 were UV irradiated on the UV barbecue, which involves placing the roller on a rotating rod circa 10 cm away from a UV source. Thus the UV barbecue irradiates the hair sample coated in polyurethane *in situ* whilst it is wound onto the roller. The following timepoints were captured: (A) hair samples post treatment but prior to immersion in the water tank below; (B) after 5 min of immersion; (C) after 1 hour of immersion; (D) after 3 hours of immersion. Conclusions drawn include: treatment with polyurethane significantly increased curl retention versus water-treated curls; treatment with UV irradiated polyurethane retained curls significantly increase curl retention compared with untreated polyurethane treatment and this effect was noticeable after treatment with 4% and 8% of crosslinked polyurethane; the effect was still clearly noticeable after 3 hours of water immersion.

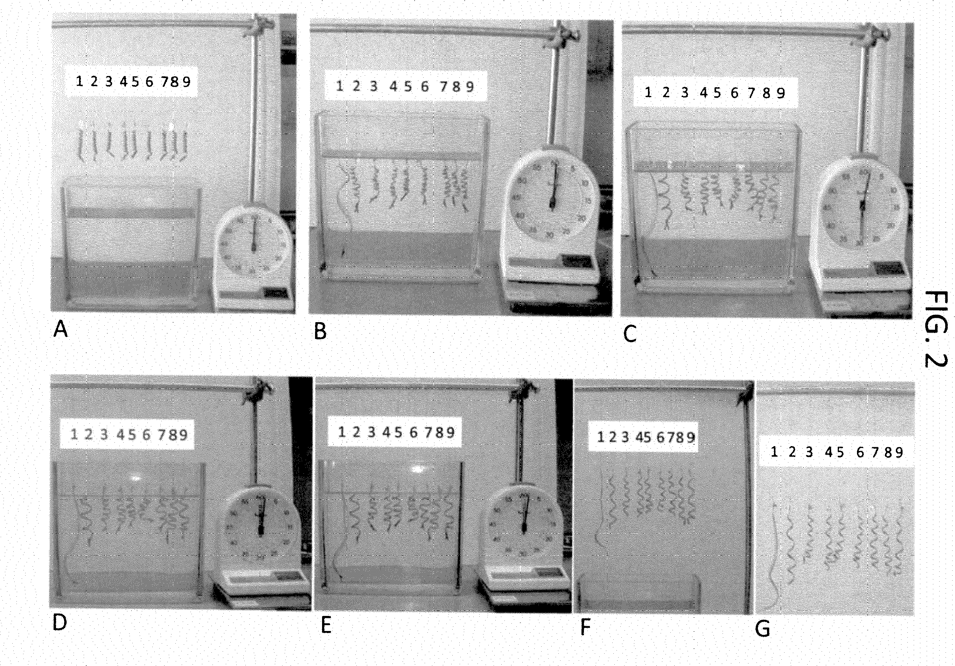

Fig. 2: Wet curl retention test - duration of UV treatment. Hair samples were treated with 16% of a polyurethane dispersion in water, which had already been exposed to UV for different time durations: from left to right (1) water only (no UV); (2) uncrosslinked polyurethane (no UV); (3) crosslinked polyurethane - 5 min UV; (4) crosslinked polyurethane - 10 min UV; (5) crosslinked polyurethane - 20 min UV; (6) crosslinked polyurethane - 30 min UV; (7) crosslinked polyurethane - 40 min UV; (8) crosslinked polyurethane - 50 min UV; (9) crosslinked polyurethane - 60 min UV. The UV irradiation of the polyurethane, prior to its application onto the hair sample (samples 3 to 9), was carried out by placing the polyurethane dispersion in water in a receptacle, which was being simultaneously vigorously stirred and irradiated with UV for a certain duration. Immediately following treatment, all the hair samples were wound onto helical rollers, dried for one hour at room temperature and then at 40°C overnight, removed from the roller, a weight added to the end of the curl and then immersed in water in order to evaluate the ability of each sample to retain the curl. The following timepoints were captured: (A) hair samples immediately prior to immersion in the water tank below; (B) after 1 min of immersion; (C) after 30 min of immersion; (D) after 120 min of immersion; (E) after 240 min of immersion; (F) after removal from the tank and removal of the weight; (G) after air drying. Conclusions drawn include: it is not necessary to UV irradiate the hair *in situ -* pre-UV irradiation of the polyurethane prior to application onto hair also results in excellent performance; the duration of UV treatment affected the amount of curl retention; the increased curl retention resultant from treatment with crosslinked polyurethane remained even after air drying the hair; irradiation for 5 min or as long as 60 min demonstrated better curl retention relative to treatment with untreated polyurethane; overall, irradiation with UV for 30 min resulted in the strongest curl retention effect.

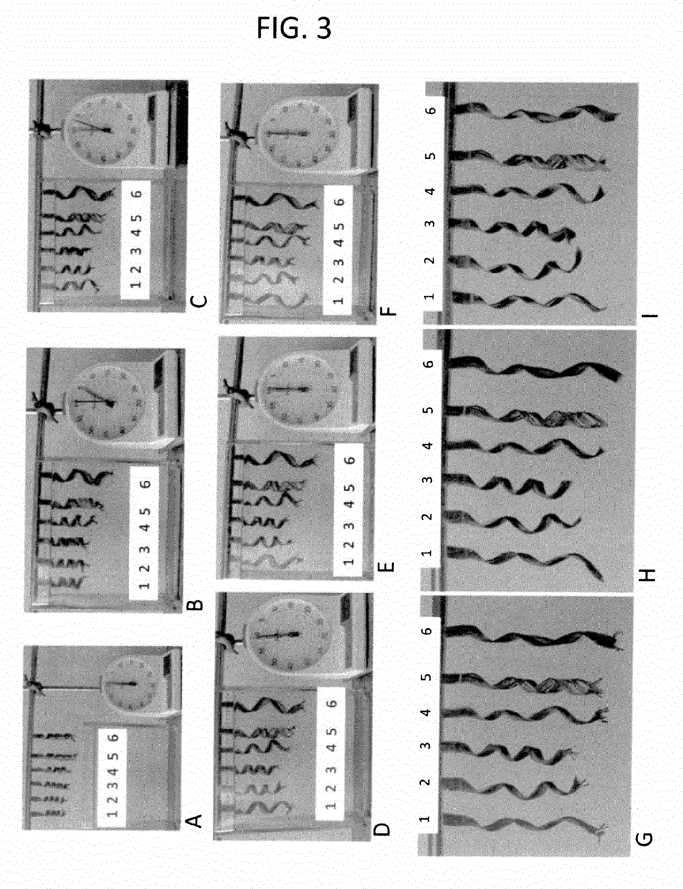

Fig. 3: Wet curl retention test - amount of cationic surfactant. Hair samples were treated with a composition comprising 6% of a crosslinked polyurethane dispersion in water (the polyurethane dispersion had been UV irradiated under agitation for 30 min) and, from left to right, the following concentrations of cetyltrimethylammonium chloride: (1) 0.00%; (2) 0.25%; (3) 0.5%; (4) 1%; (5) 2%; (6) 4%. Immediately following treatment, the hair samples were wound onto helical rollers, dried for one hour at room temperature and then at 40°C overnight, removed from the roller, a weight (2 x 50 mg) added to the end of the curl and then immersed in water in order to evaluate the ability of each sample to retain the curl. The following timepoints were captured: (A) hair samples immediately prior to immersion in the water tank below; (B) after 5 min of immersion; (C) after 60 min of immersion; (D) after 120 min of immersion; (E) after 240 min of immersion; (F) after 21 hours of immersion; (G) after removal from the tank; (H) after removal of the weight; (I) after air drying. The same hair samples were also ranked according to hair feel - good visual appearance including, for example, lack of stickiness, lack of stringiness, lack of unnatural or unpleasant hair feel, good manageability. Relative to each other from best to worst, the ranking vis-à-vis hair feel was as follows: sample (3); sample (2); sample (1); sample (4); sample (6); sample (5). The same samples were also ranked according to visual appearance - good visual appearance including, for example, lack of clumping together of hair strands. Relative to each other from best to worst, the ranking vis-à-vis hair appearance was as follows: sample (3); sample (2); sample (4); sample (1); sample (6); sample (5). Conclusions drawn include: treatment of hair samples with both crosslinked polyurethane and cationic surfactant can significantly improve hair curl retention versus hair samples treated with crosslinked polyurethane alone; this effect is still obvious even after 21 hours of water immersion and also after air drying; treatment with 0.5% of the cationic surfactant in combination with crosslinked polyurethane resulted in the best curl retention, hair feel and hair appearance versus the other treatment conditions tested.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A composition comprising polymer P^{x}, wherein polymer P^{x} comprises a moiety of formula selected from the group consisting of: Formula 1, Formula 2 and a combination of Formula 1 and Formula 2: 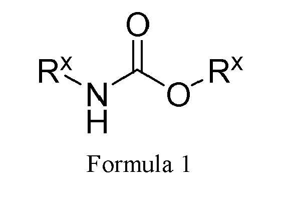 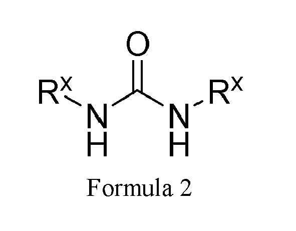 wherein R^{x} comprises a group according to Formula 3: 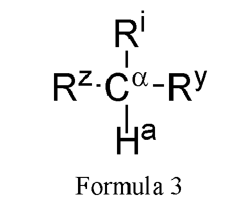 wherein R^{y} and R^{z} are, independently, any suitable atom or moiety; and Rⁱ is an electron-withdrawing functional group;
and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

2. A composition comprising polymer P^{x}, wherein polymer P^{x} comprises a moiety of formula selected from the group consisting of Formula 4 and Formula 5: 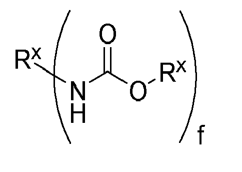 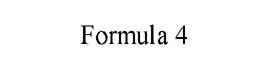 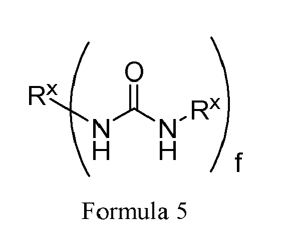 wherein R^{x} comprises a group according to Formula 3: 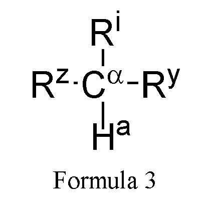 wherein R^{y} and R^{z} are, independently, any suitable atom or moiety; and Rⁱ is an electron-withdrawing functional group;
and wherein f is from 4 to 40;
and wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

3. A composition comprising a dispersed polyurethane comprising a polymer P^{x} which is the reaction products of:
A) a prepolymer according to the Formula PP: 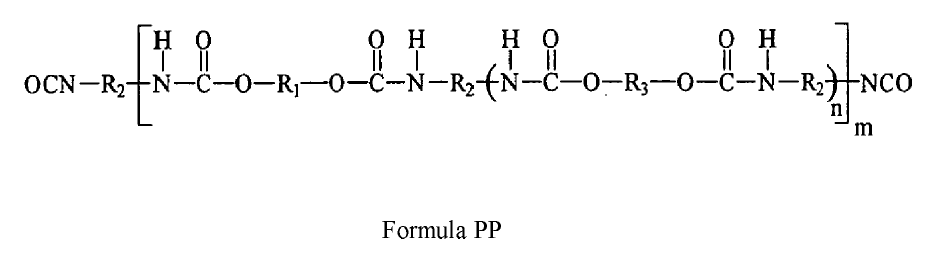 wherein
R₁ represents a bivalent radical of a dihydroxyl functional compound, R₂ represents a hydrocarbon radical of an aliphatic or cycloaliphatic polyisocyanate,
R₃ represents a radical of a low molecular weight diol, optionally substituted with ionic groups,
n is from 0 to 5, and m is >1;
B) at least one first chain extender according to the formula: H₂N-R₄-NH₂ wherein R₄ represents an alkylene or alkylene oxide radical not substituted with ionic or potentially ionic groups; and
C) at least one second chain extender according to the formula: H₂N-R₅-NH₂ wherein R₅ represents an alkylene radical substituted with ionic or potentially ionic groups;
wherein at least one of R₁, R₂ and R₃ comprises a group according to Formula 3, as recited in any preceding claim;
wherein polymer P^{x} comprises at least one direct crosslink, wherein the direct crosslink is a direct covalent bond between the C^{α} of the polymer P^{x} and a second C^{α} of a polymer P^{x}, and wherein said direct crosslink comprises no intermediary atom.

4. The composition, according to any of the preceding claims, wherein Rⁱ comprises an electron-withdrawing functional group selected from the group consisting of: carboxyl; thiocarboxyl; carbonyl; thiocarbonyl; imine; hydroxyl; aryl; heteroaryl; a saturated cyclic radical; alkoxy; amino; amide; imide; phosphorus; sulphur; tin; silica; and alkyne.

5. A cosmetic composition comprising:
(a) the composition, according to any of the preceding claims; and
(b) a cosmetically acceptable carrier.

6. The cosmetic composition, according to claim 5, wherein the cosmetically acceptable carrier is water and the composition comprises from about 0.001% to about 10%, preferably from about 0.1% to about 9.0%, more preferably from about 2.0% to about 8.0%, even more preferably from about 3% to about 7.0%, most preferably from about 4.0% to about 6.0% of polymer P^{x}, by total weight of the cosmetic composition.

7. The composition, according to any preceding claim, further comprising a reaction product of a photoinitiator, wherein the reaction product of a photoinitiator is selected from the group consisting of: ammonium hydrogensulfate; sodium hydrogensulfate; potassium hydrogensulfate; lithium hydrogensulfate; and mixtures thereof.

8. A method of styling hair comprising:
(a) providing a composition according to any of claims 1 to 7;
(b) applying said composition to hair;
(c) styling the hair.

9. The use of a composition, according to any of claims 1 to 7, for styling hair.

10. A kit comprising:
(a) a cosmetic formulation comprising:
i. a polymer P comprising a moiety of formula selected from the group consisting of: Formula 1, Formula 2, and a combination of Formula 1 and Formula 2: 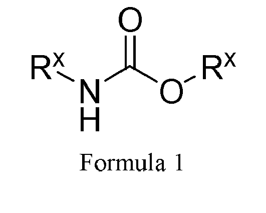 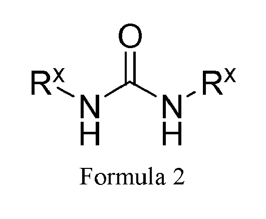 wherein R^{x} comprises a group according to Formula 3: 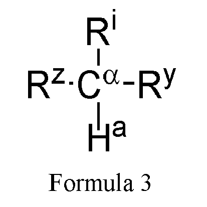 and wherein R^{y} and R^{z} are, independently, any suitable atom or moiety; and
Rⁱ is an electron-withdrawing functional group;
ii. a cosmetically acceptable carrier;
(b) a photoinitiator;
wherein polymer P is capable of forming at least one direct crosslink, wherein the direct crosslink is a direct covalent bond between the Cα of the polymer P and a second C^{α} of a polymer P, and wherein said direct crosslink comprises no intermediary atom.

11. The kit, according to claim 10, further comprising:
(e) a device for irradiating the cosmetic formulation with UV;
(f) instructions.

12. The kit, according to any of claims 10 to 11, further comprising a stirring means.

13. A method of styling hair comprising:
I. providing the kit, according to any of claims 10 to 12;
II. mixing the cosmetic formulation with the photoinitiator resulting in a mixed cosmetic formulation;
III. irradiating said mixed cosmetic formulation with UV resulting in an irradiated cosmetic formulation;
IV. applying the irradiated cosmetic formulation to hair;
V. styling the hair;
VI. exposing the hair to a temperature of from about 20°C to about 80°C, preferably from about 30°C to about 60°C, more preferably from about 35°C to about 55°C, even more preferably from about 40°C to about 50°C.

14. A process comprising:
(a) the provision of a formulation comprising a polymer P, wherein polymer P comprises a moiety of formula selected from the group consisting of: Formula 1, Formula 2, and a combination of Formula 1 and Formula 2: 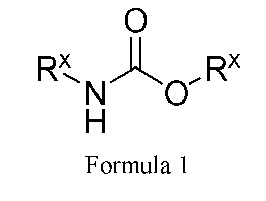 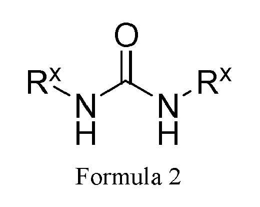 wherein R^{x} comprises a group according to Formula 3: 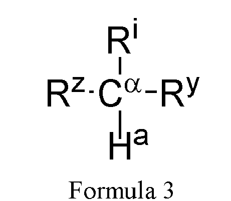 and wherein R^{y} and R^{z} are, independently, any suitable atom or moiety; and Rⁱ is an electron-withdrawing functional group;
(b) the provision a photoinitiator;
(c) mixing said polymer P with said photoinitiator to form a mixed formulation;
(d) the irradiation of said mixed formulation with UV to form at least one direct crosslink, wherein the direct crosslink is a direct covalent bond between the C^{α} of the polymer P and a second C^{α} of a polymer P, and wherein said direct crosslink comprises no intermediary atom.

15. A process, according to claim 14, wherein during the irradiation, the formulation is under vigorous agitation.
